# EUROPEAN PATENT SPECIFICATION

(11) **EP 2 767 214 B1**
(45) Date of publication and mention of the grant of the patent: **19.07.2017**
(21) Application number: 13845418.6
(22) Date of filing: 26.09.2013
(51) Int. Cl.: A61B 1/00, A61B 8/12, A61B 10/02

(54) **TREATMENT INSTRUMENT**
BEHANDLUNGSINSTRUMENT
INSTRUMENT DE TRAITEMENT

(30) Priority: 10.10.2012 JP 2012225303
(43) Date of publication of application: 20.08.2014
(73) Proprietor: Olympus Corporation, Tokyo 192-8507 (JP)
(72) Inventor: OGAWA Tomoaki, Tokyo 192-8507 (JP)
(74) Representative: Schicker, Silvia
(86) International application number: PCT/JP2013/076029
(87) International publication number: WO 2014/057813

(56) References cited:
- EP-A1- 2 883 503
- JP-A- 2011 528 962
- JP-U- H0 541 509
- US-A- 5 217 450
- US-A- 5 571 136
- US-A1- 2006 074 343
- US-A1- 2006 149 222

## Description

### Technical Field

The present invention relates to a treatment instrument that endoscopically collects a sample.

### Background Art

Conventionally, for peripheral lesions in the respiratory field, for example, a small lesion of, e.g., a lung periphery, observation and diagnosis, and sample collection are performed using, e.g., a bronchoscope; however, it is difficult to perform reliable diagnosis and sample collection by means of visual observation alone. Therefore, in recent years, more reliable diagnosis is performed by, for example, collecting a sample of a lesion part by means of a guide sheath method using a bronchoscope for peripheries and analyzing the collected sample.

Here, as disclosed in, for example, Japanese Patent Application Laid-Open Republication No. 2007/055032, the guide sheath method is a method as follows: after the position of a lesion is confirmed fluoroscopically, for example, a small-diameter ultrasound probe is bronchoscopically guided to the peripheral lesion to confirm, e.g., the position of the lesion and the state of the lesion via an ultrasound image. Subsequently, with a distal end of the guide sheath positioned in the vicinity of the peripheral lesion, a treatment instrument such as biopsy forceps is inserted replacing the inserted ultrasound probe. Then, a sample of cells or a tissue of the lesion is collected using the treatment instrument such as biopsy forceps. The sample collected as described above is analyzed to perform a disease diagnosis.

In a case where a biopsy target is located at a small-diameter portion, there is a case where an object of an intended amount cannot be collected at a time in dependence on a shape of the treatment instrument and the insertion has to be performed repeatedly.

Further, document US 5,217,450 A discloses a retention device which can be removably retained in a body cavity. The retention device comprises branch members which are movably connected to each other such that two relative configurations may be obtained. In one configuration the branch members are nested inside one another for inserting the retention device in a cavity. Once in a cavity an operating member is used to displace the branch members to form a second configuration, in which parts of the branch members are expanded to bear against the walls of the cavity and retain the device.

The present invention has been made in view of the foregoing points and an object of the present invention is to provide a treatment instrument for collecting a sample which is capable of securely performing a treatment of collecting a sample of a peripheral lesion or the like in a short time.

### Disclosure of Invention

### Means for Solving the Problem

A treatment instrument according to the present invention is defined in claim 1. Preferred embodiments are defined in the dependent claims. According to the present invention, it is possible to provide a treatment instrument for collecting a sample which is capable of securely performing a treatment of collecting a sample of a peripheral lesion or the like.

### Brief Description of the Drawings

Fig. 1 includes diagrams illustrating an overview of a configuration and an operation of a treatment instrument;
Fig. 2 is an enlarged main part diagram illustrating an enlargement of only a link portion and an advancing/retracting portion extracted from a sample collection portion in the treatment instrument;
Fig. 3 is an enlarged main part diagram illustrating a first modification of the sample collection portion in the treatment instrument;
Fig. 4 is an enlarged main part diagram illustrating a second modification of the sample collection portion in the treatment instrument;
Fig. 5 is an enlarged main part diagram illustrating a third modification of the sample collection portion in the treatment instrument;
Fig. 6 is a cross-sectional diagram illustrating an overview of a configuration of a treatment instrument according to an embodiment of the present invention;
Fig. 7 is a diagram illustrating a schematic configuration of an operation portion in the treatment instrument according to the embodiment of the present invention;
Fig. 8 includes diagrams illustrating an overview of an operation of the treatment instrument according to the embodiment of the present invention;
Fig. 9 is a cross-sectional diagram illustrating an overview of a configuration of a modification of the treatment instrument according to the embodiment of the present invention;
Fig. 10 is a cross-sectional diagram illustrating an overview of a configuration of a treatment instrument according to a second embodiment of the present invention;
Fig. 11 is a top view illustrating only a sample collection portion extracted from the treatment instrument according to the second embodiment of the present invention;
Fig. 12 is a side view of only the sample collection portion extracted from the treatment instrument according to the second embodiment of the present invention; and
Fig. 13 is a diagram illustrating a schematic configuration of a modification of a treatment instrument.

### Best Mode for Carrying Out the Invention

The present invention will be described below by means of the illustrated embodiments. Note that, in the respective drawings used for the below description, respective components may be illustrated on different scales in order to indicate the respective components in respective sizes that can be recognized on the drawings. Accordingly, in the present invention, the counts and quantities of the components illustrated in the drawings, the shapes of the components, the ratios in size among the components and relative positional relationships among the respective components are not limited to those of the forms illustrated in the drawings.

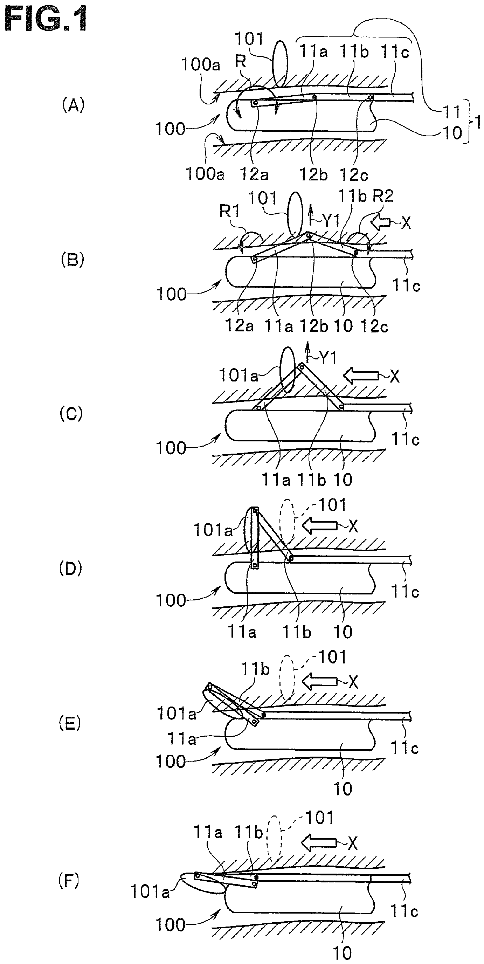

Fig. 1 includes diagrams illustrating an overview of a configuration and an operation of a treatment instrument. A treatment instrument 1 includes an insertion portion 10 that is formed in a rod-like shape and can be inserted into a lumen 100 of a subject, and at least one sample collection portion 11 arranged on a circumferential face of the insertion portion 10.

The sample collection portion 11 includes a link portion 11 a, an advancing/retracting portion 11b and a push rod 11c, which are connected in series. An end 12a of the link portion 11a is fixedly provided on the outer circumferential face of the insertion portion 10. In this case, the link portion 11a is disposed on the insertion portion 10 in such a manner that the link portion 11a is pivotable in the arrow R direction with the end 12a as a central axis of rotation. The configuration makes the link portion 11a be supported on the insertion portion 10 in such a manner that an end (12a) of the link portion 11a serves as a rotation axis and the other end (12b) is pivotable in an axial direction of the insertion portion 10.

An end 12b of the advancing/retracting portion 11b is joined to the other end 12b of the link portion 11a in such a manner that the link portion 11a and the advancing/retracting portion 11b are pivotable relative to each other. A joint 12c at an end of the push rod 11c is joined to the other end 12c of the advancing/retracting portion 11b in such a manner that the advancing/retracting portion 11b and the push rod 11c are pivotable relative to each other. With this configuration, the advancing/retracting portion 11b is joined to the other end of the link portion 11a, and thus, the push rod 11c is advanced/retracted in the axial direction, whereby the other end 12b of the link portion 11a pivots in the axial direction. Provision of a structure such as described above enables a subject to be hollowed using the 11a part to collect a target object. Depending on the length and/or the width of 11a, a larger amount of target object can be collected at a time compared to treatment instruments that scrape a subject for biopsy and treatment instruments that pinch a subject for biopsy.

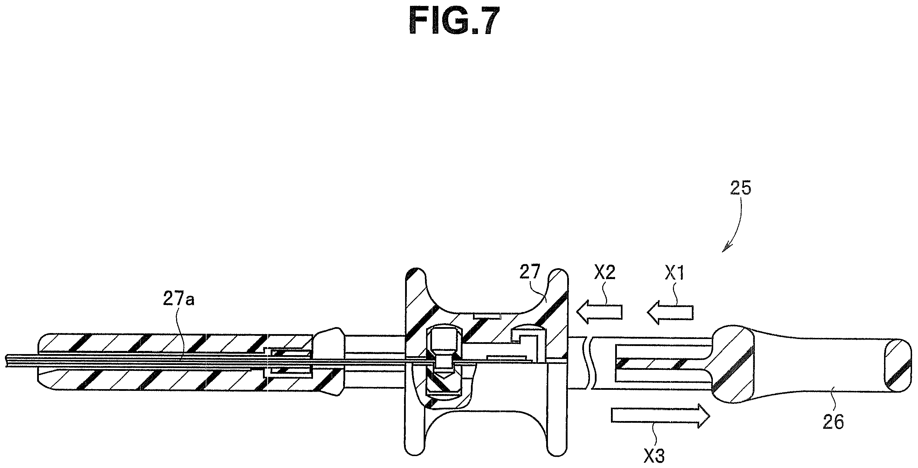

The push rod 11c is disposed so that the push rod 11c is not spaced from the outer circumferential face of the insertion portion 10 and can be advanced/retracted along the axial direction (X direction) of the insertion portion 10. At the other end (not illustrated in Fig. 1) of the push rod 11c, an operation portion (see Fig. 7 described later) is disposed. The operation portion is a member that advances/retracts the push rod 11c in a direction along the axial direction (X direction) of the insertion portion 10.

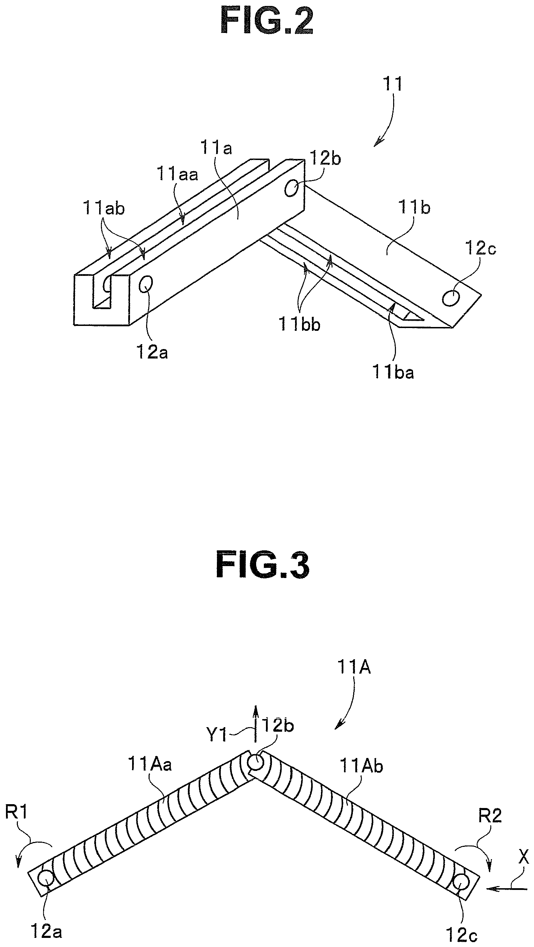

Also, Fig. 2 is an enlarged diagram of essential parts, which illustrates an enlargement of only the link portion 11a and the advancing/retracting portion 11b extracted from the sample collection portion in the treatment instrument 1. Also, Figs. 3, 4 and 5 each illustrate a modification of the sample collection portion in the treatment instrument 1. As illustrated in Fig. 2, each of the link portion 11a and the advancing/retracting portion 11b of the sample collection portion in the treatment instrument 1 is created by, for example, folding, e.g., a thin plate-like metal member to form a recess portion 11aa or 11ba having a recess shape in cross-section. In this case, the recess portion 11aa of the link portion 11a is formed so that an opening thereof faces outward in an assembled state. On the other hand, the recess portion 11ba of the advancing/retracting portion 11b is formed so that an opening thereof faces inward, that is, faces the outer circumferential face of the insertion portion 10 also in an assembled state. Each of opening edge portions 11ab and 11bb of the link portion 11a and the advancing/retracting portion 11b is formed in a sharp shape. Since Fig. 2 is an enlarged diagram, the sharpness of the opening edge portions 11ab and 11bb is not expressed; however, in reality, the opening edge portions 11ab and 11bb each have an extremely small width dimension and thus have sharpness. As described above, the opening edge portions 11ab and 11bb have sharpness, thereby each forming a scraping portion that can scrape a lesional tissue, which is a target site.

Note that Figs. 3, 4 and 5 illustrate respective modifications of the link portion and the advancing/retracting portion of the sample collection portion in the treatment instrument. A sample collection portion 11A according to a first modification, which is illustrated in Fig. 3, has a form in which a link portion 11Aa and an advancing/retracting portion 11Ab are each formed in, for example, a round rod shape and a helical thread portion is formed at an outer circumferential face of each of the link portion 11Aa and the advancing/retracting portion 11Ab. In the sample collection portion 11A having this form, when the advancing/retracting portion 11Ab receives an amount of force in the arrow X direction in Fig. 3, the link portion 11Aa pivots in the arrow R1 direction with an joint 12a at one end thereof as a rotation axis, the advancing/retracting portion 11Ab pivots in the arrow R2 direction with the joint 12c at the other end thereof as a rotation axis, and a joint 12b between the link portion 11Aa and the advancing/retracting portion 11Ab moves in the arrow Y1 direction. In this case, when a surface (the helical thread portion) of the sample collection portion 11A (the link portion 11Aa and the advancing/retracting portion 11Ab) slides in contact with a lesion part, a tissue of the lesion part can be collected.

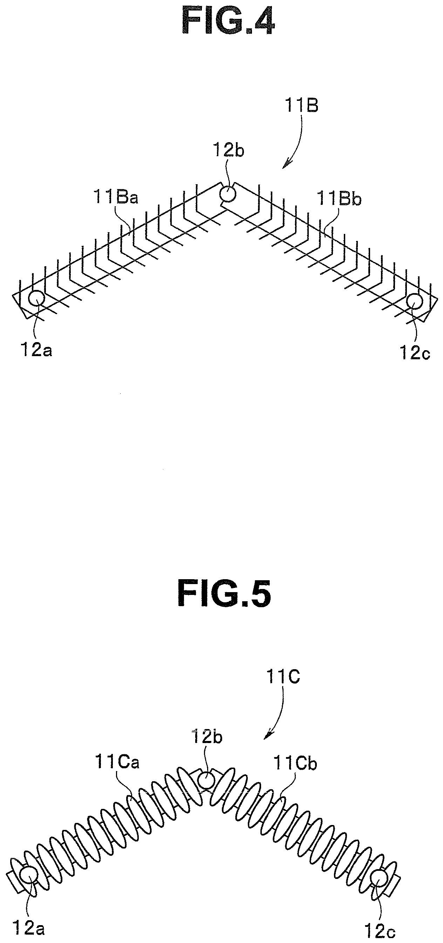

A sample collection portion 11B according to a second modification, which is illustrated in Fig. 4, has a form in which a link portion 11Ba and an advancing/retracting portion 11Bb are each formed in, for example, a round rod shape and a brushlike brush portion is disposed on an outer circumferential face of each of the link portion 11Ba and the advancing/retracting portion 11Bb. The sample collection portion 11B having this form provides an operation substantially similar to those of the sample collection portions according to the embodiment and the first modification described above. Consequently, when the brush portion included in a surface of the sample collection portion 11B (the link portion 11Ba and the advancing/retracting portion 11Bb) slides in contact with a lesion part, a tissue of the lesion part can be collected. The brush portion may be one formed by bristle implantation or one formed by integral molding.

A sample collection portion 11C according to a third modification, which is illustrated in Fig. 5, has a form in which a link portion 11Ca and an advancing/retracting portion 11 Cb are each formed in, for example, a round rod shape and a bellows-like recess and projection portion is formed or abacus beads are aligned on an outer circumferential face of each of the link portion 11Ca and the advancing/retracting portion 11Cb. The sample collection portion 11C having this form also provides an operation substantially similar to those of the sample collection portions according to the first and second modifications described above. Consequently, when a surface (the bellows-like recess and projection portion) of the sample collection portion 11C (the link portion 11Ca and the advancing/retracting portion 11Cb) slides in contact with a lesion part, a tissue of the lesion part can be collected. An overview of an operation of the treatment instrument 1 configured as described above is described below.

First, the state illustrated in Fig. 1(A) is a state in which the sample collection portion 11 in the treatment instrument 1 according to the present embodiment is in a housed state and is inserted inside a lumen 100 of a subject.

In this state, the sample collection portion 11 in the treatment instrument 1 is disposed along the outer circumferential face of the insertion portion 10 in such a manner that the link portion 11a, the advancing/retracting portion 11b and the push rod 11c are continuously provided in this order from the distal end side, forming a linear shape in its entirety. In other words, this shape is convenient for insertion into a lumen 100 of a subject. In other words, also, in this state, the advancing/retracting portion 11b exists on the most proximal end side, and the advancing/retracting portion 11b and the link portion 11a are connected along the axial direction, and disposed parallel to the insertion portion 10 in the axial direction.

As illustrated in Fig. 1(A), the treatment instrument 1 in this state is inserted to, for example, a small-diameter lumen 100 such as a bronchus of a subject, and a part around a distal end portion of the treatment instrument 1 is arbitrarily advanced and guided to the inside. Then, the part around the distal end portion of the treatment instrument 1 is fixedly arranged in the vicinity of a lesion part 101 whose position has been identified in advance in the subject.

In this state, for example, a user performs a predetermined operation of the operation portion (not illustrated) to move the push rod 11c in the treatment instrument 1 in an axial direction of the treatment instrument 1, that is, the arrow X direction indicated in Fig. 1(B). Then, as illustrated in Fig. 1(B), the link portion 11a pivots in the arrow R1 direction with the end 12a thereof as a rotation axis. Simultaneously, the advancing/retracting portion 11b is pushed by the push rod 11c and thereby pivots in the arrow R2 direction with the joint 12c as a rotation axis. Consequently, the joint 12b between the link portion 11a and the advancing/retracting portion 11b moves in the arrow Y1 direction so as to move away from the outer circumferential face of the insertion portion 10. Consequently, the link portion 11a and the advancing/retracting portion 11b eventually cut through an inner wall face 100a and reach a position where the link portion 11a and the advancing/retracting portion 11b come into touch with a part of the lesion part 101 (the state illustrated in Fig. 1(C)).

As described above, during a period from the state illustrated in Fig. 1(B) to the later-described state illustrated in Fig. 1(D) through the state illustrated in Fig. 1(C), that is, when the advancing/retracting portion 11b is located between the proximal end portion side and the distal end portion side, the link portion 11a is pushed out in a direction crossing the axial direction of the insertion portion 10 (the arrow Y1 direction) by the advancing/retracting portion 11b pushed by the push rod 11c and thereby projects in a direction away from the outer surface of the insertion portion 10.

When the push rod 11c is further moved in the arrow X direction from the state illustrated in Fig. 1(C), the link portion 11a is gradually moved away from the outer surface of the insertion portion 10, and when the state illustrated in Fig. 1(D) is reached, the link portion 11a projects so as to erect on the insertion portion 10. The advancing/retracting portion 11b also gradually moves away from the outer surface of the insertion portion 10 and projects following the link portion 11a, and the state illustrated in Fig. 1(E) is reached and the advancing/retracting portion 11b begins overlapping the link portion 11a. In this process, the recess portion 11aa of the link portion 11a scrapes a lesional tissue 101a of the lesion part 101 off. Also, the recess portion 11ba of the advancing/retracting portion 11b also scrapes a lesional tissue 101a of the lesion part 101 off.

Then, when the state illustrated in Fig. 1(E) transitions to the state illustrated in Fig. 1(F), the advancing/retracting portion 11b is arranged on the most distal end side, and the link portion 11a and a part of the advancing/retracting portion 11 b are connected in an overlapped manner and disposed parallel to the insertion portion 10. At this time, the advancing/retracting portion 11b is located on the most distal end side and in this state, the other end (the joint 12b) of the link portion 11a is arranged on the distal end side relative to a most distal end face of the insertion portion 10. Note that the scraped lesional tissue 101a reliably adheres to the recess portion 11aa of the link portion 11a.

After the collection of the lesional tissue 101a from the lesion part 101 as described above, an operation to pull the treatment instrument 1 out with the sample collection portion 11 kept in the closed state illustrated in Fig. 1(F) is performed. As described above, use of the treatment instrument 1 enables reliable collection of a subject from a desired site.

As described above, according to the first embodiment, the sample collection portion 11 is disposed parallel to the axial direction on the outer circumferential face of the insertion portion 10 until the sample collection portion 11 reaches a lesion part, and thus, it is easy to insert the treatment instrument 1 to a very narrow lumen and guide the treatment instrument 1 to a lesion part, for example, in the case of a lesion of a lung periphery.

When a lesional tissue of a lesion part is collected using the treatment instrument 1 that has reached the vicinity of the lesion part, for example, even if the lesion part exists outside a wall of, e.g., a bronchus, the lesional tissue can be collected by extending the sample collection portion 11 from the position inside the lumen toward the outside of the wall. Accordingly, lesional tissue collection can be performed easily and reliably as described above, enabling contribution to diagnostic yield enhancement.

Also, a larger amount of lesional tissue can be collected by taking measures to make the sample collection portion 11 include a shape suitable for scraping a lesional tissue off, that is, e.g., a shape provided with recess portions 11aa and 11ba, a helical thread portion (the first modification), a brush portion (the second modification) or a bellows-like recess and projection portion (the third modification).

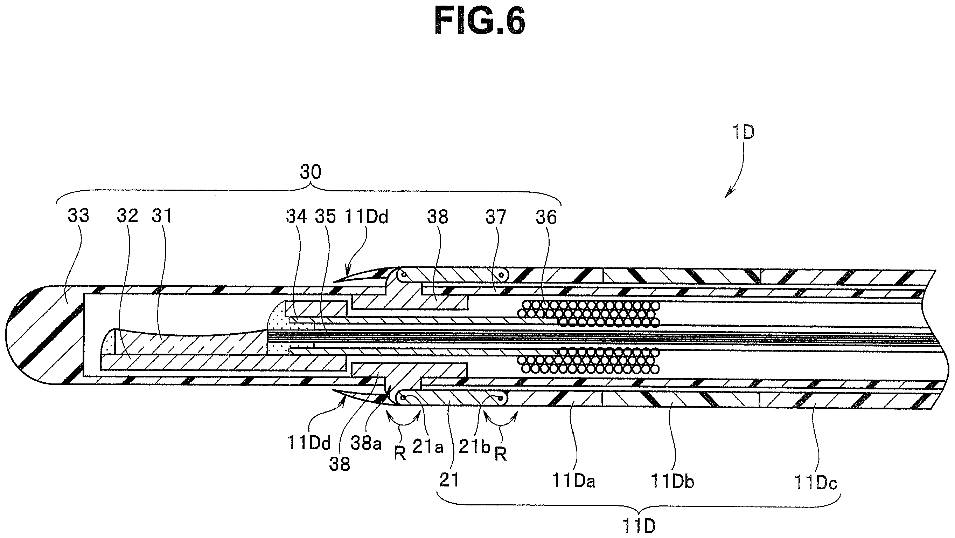

Fig. 6 is a cross-sectional diagram illustrating an overview of a configuration of a treatment instrument according to an embodiment of the present invention.

As illustrated in Fig. 6, a treatment instrument 1D according to the present embodiment is an example in which, for example, a mechanical radial scanning-type small-diameter ultrasound probe 30 and a plurality of (two in the present embodiment) sample collection portions 11D are integrally formed. Here, the ultrasound probe 30 is a component member corresponding to the insertion portion in the first described instrument. The ultrasound probe 30 mainly includes, e.g., an ultrasound transducer 31, a housing 32, a distal end cap 33, a small-diameter shaft 34, an ultrasound cable 35, a flexible shaft 36, a sheath 37, and a sleeve 38.

The ultrasound transducer 31 is an ultrasound transmission/reception section for generating ultrasound, and is held by the housing 32. The housing 32 is a casing component that fixedly holds respective surfaces of the ultrasound transducer 31 except an ultrasound transmission/reception surface of the ultrasound transducer 31.

Note that the ultrasound transmission/reception section of the ultrasound probe 30 in the present embodiment is disposed so as to transmit/receive ultrasound in a direction perpendicular to an axial direction.

The distal end cap 33 includes a most distal end part having a substantially hemispherical shape in cross section, and behind the hemispherical shape portion, a substantially cylindrical shape portion is provided so as to be continuous with the hemispherical shape portion. Behind the cylindrical shape portion of the distal end cap 33, and the tubular sheath 37 is provided so as to be continuous with the cylindrical shape portion via the sleeve 38. In an inner space of the distal end cap 33, the ultrasound transducer 31 fixedly held by the housing 32 is housed in such a manner that the ultrasound transducer 31 is pivotable around the axis. Also, the inside of the distal end cap 33 is charged with an ultrasound medium. The housing 32 is fixedly provided at a distal end of the small-diameter shaft 34. A distal end of the flexible shaft 36 is fixedly provided at a distal end of the small-diameter shaft 34. The ultrasound cable 35 is inserted through inner cavities of the small-diameter shaft 34 and the flexible shaft 36. The ultrasound cable 35 extends from the ultrasound transducer 31 and connects the ultrasound transducer 31 and an ultrasound control apparatus (not illustrated) disposed on the hand side. The small-diameter shaft 34 and the flexible shaft 36 are inserted and arranged inside the sheath 37.

The sleeve 38 serves to connect the distal end cap 33 and the sheath 37 as described above and also serves to fixedly hold parts of the sample collection portions 11D as described later. For that purpose, in the sleeve 38, projection portions 38a projecting in a radial direction perpendicular to an axial direction of the treatment instrument 1D are formed, and an end 21a of a link arm 21 is attached to each of the projection portions 38a in such a manner that the link arm 21 is pivotable in the arrow R direction in Fig. 6. A part around a middle of a cell collection link portion 11Da (which will be described later) of each of the sample collection portions 11D is attached to the other end 21 b of the corresponding link arm 21 in such a manner that the part is pivotable in the arrow R direction in Fig. 6, thereby forming a link mechanism.

Each of the sample collection portions 11D in the treatment instrument 1D according to the present embodiment mainly includes the cell collection link portion 11Da, an advancing/retracting portion 11Db, which is flexible, and a push rod 11Dc.

The sample collection portions 11D are disposed on an outer circumferential face of parts on the distal end side of the ultrasound probe 30. In this case, the sample collection portions 11D are disposed at positions where the sample collection portions 11D do not cover an outer face of the distal end cap 33, in a housed state. In other words, the sample collection portions 11D are disposed at respective sites where the sample collection portions 11D do not interfere with a range of ultrasound observation via the ultrasound probe 30.

A most distal end portion of each cell collection link portion 11Da is formed so as to have a sharp shape and includes a bent scraping portion 11Dd having a shape that bends toward the inside. Note that, here, the "inside" refers to a side facing an outer circumferential face of the ultrasound probe 30 in a housed state in which the relevant sample collection portion 11D is attached to the treatment instrument 1D. The other end 21b of the link arm 21 is pivotably attached to the part around the middle of the cell collection link portion 11Da.

Note that the link arms 21 and the cell collection link portions 11Da are formed by, e.g., a metal or a rigid resin, for example, a PEEK (polyetheretherketone) resin.

An end of the advancing/retracting portion 11Db having flexibility is integrally formed on the proximal end side of each cell collection link portion 11Da. The advancing/retracting portion 11Db is formed by, e.g., a flexible resin or a metal coil.

The push rod 11Dc is integrally formed on the proximal end side of the advancing/retracting portions 11Db. The push rod 11Dc is formed by a tubular member that covers the sheath 37 of the ultrasound probe 30, and the proximal end side thereof extends up to a non-illustrated operation portion. The push rod 11Dc is formed by, e.g., a tubular resin member or a metal coil.

Note that in the treatment instrument 1D according to the present embodiment, two sample collection portions 11D are provided. In such a case, the sample collection portions 11D are arranged at positions facing each other with an angle of around 180 degrees therebetween in a radial direction of the ultrasound probe 30. However, the present invention is not limited to such configuration, and only one sample collection portion 11D may be provided, or three or more sample collection portions 11D may be provided. When a plurality of sample collection portions 11D are provided, it is common to provide the sample collection portions 11D at even intervals in a radial direction of a probe as an arrangement thereof; however, it should be understood that the present invention is not limited to such arrangement.

Next, a schematic configuration of an operation portion 25 in the treatment instrument 1D according to the present embodiment will be described with reference to Fig. 7.

The operation portion 25 in the treatment instrument 1D according to the present embodiment includes a grasping portion 26 and a push rod handle 27. The grasping portion 26 is a part that holds the push rod handle 27 in such a manner that the push rod handle 27 is slidable in an axial direction, and is grasped with, e.g., the hands by a user at the time of use. The push rod handle 27 is an operation member that performs an operation to open/close the sample collection portions 11D by moving the push rod handle 27 in the axial direction to push/pull the push rod 11Dc of the sample collection portion 11D. Thus, the push rod handle 27 and the push rod 11Dc of the sample collection portions 11D are connected via a wire 27a. Accordingly, when the push rod handle 27 is pushed to the insertion portion side in the arrow X1 direction in Fig. 7, the push rod 11Dc is pushed and the sample collection portions 11D are displaced in an open direction. On the other hand, when the push rod handle 27 is pulled to the proximal end side in the arrow X3 direction in Fig. 7, the push rod 11Dc is also pulled and the sample collection portions 11D are disposed in a close direction (detailed description of the operation will be provided later).

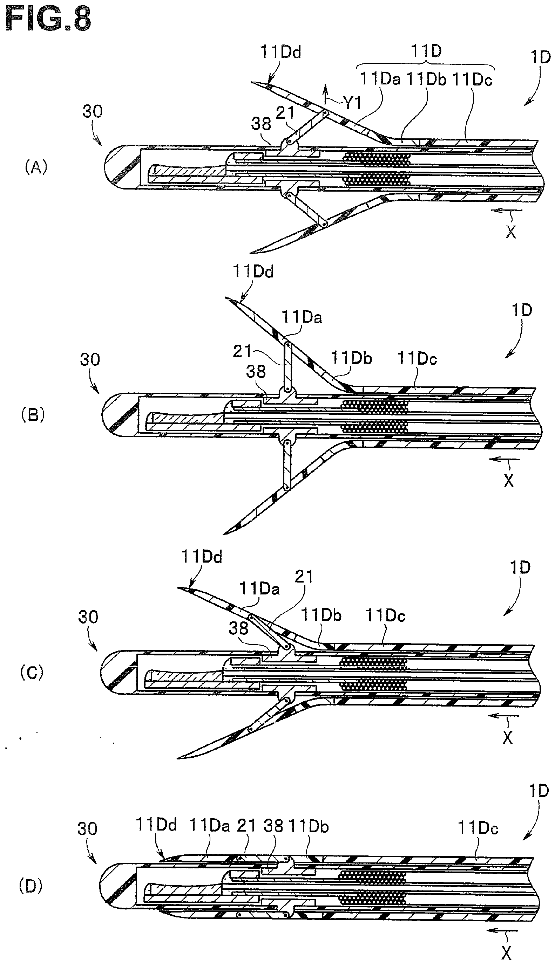

An overview of an operation of the treatment instrument 1D according to the present embodiment configured as described above will be described below with reference to Figs. 7 and 8.

First, the state illustrated in Fig. 7 indicates a state in which the sample collection portions 11D in the treatment instrument 1D according to the present embodiment are housed. Each of the sample collection portions 11D in the treatment instrument 1D in this state is provided along the outer circumferential face of the ultrasound probe 30 in such a manner that the cell collection link portion 11Da, the link arm 21, the advancing/retracting portion 11Db and the push rod 11Dc are continuously provided in this order from the distal end side, forming a linear shape in its entirety.

After the treatment instrument 1D in this state is endoscopically inserted to a desired examination site, for example, a bronchus periphery and guided to a vicinity of a lesion part, ultrasound scanning via the ultrasound probe 30 is started to search for the lesion part.

If the lesion part is caught by means of the ultrasound scanning via the ultrasound probe 30, an operation to push the push rod handle 27 in the axial direction (insertion direction: the arrow X1 direction in Fig. 7) is performed with the ultrasound probe 30 kept at a position where the lesion part has a largest diameter. Consequently, the push rod 11Dc is pushed in the axial direction along the outer circumferential face of the ultrasound probe 30.

Then, the push rod 11Dc pushes the cell collection link portions 11Da in a same direction as above via the advancing/retracting portions 11Db. Each of the cell collection link portions 11 Da is pivotably supported by the corresponding link arm 21 at the part around the middle thereof. Accordingly, as illustrated in Fig. 8(A), each of the cell collection link portions 11Da is extended by the corresponding link arm 21 in a direction in which the cell collection link portion 11Da projects in the radial direction of the ultrasound probe 30 while moving forward. Consequently, the distal end part of the cell collection link portion 11Da is pushed into the lesion part. Then, when the link arms 21 are in a substantially erected state as illustrated in Fig. 8(B), the cell collection link portions 11Da are at respective largest opened positions.

When the push rod handle 27 is further pushed in the arrow X2 direction in Fig. 7 from this state, the cell collection link portions 11Da are displaced to their respective closed positions while advancing forward as illustrated in Fig. 8(C), and finally, are arranged on parts on the distal end side of the ultrasound probe 30 along the outer circumferential face of the ultrasound probe 30 as illustrated in Fig. 8(D).

As described above, each of the distal ends of the cell collection link portions 11Da is formed so as to be sharp and form a shape that bends toward the inside. Therefore, through the operation described above, the bent scraping portions 11Dd having a bent shape at the distal ends of the cell collection link portions 11Da widely hollow and scrape a lesion part off, whereby cells or a tissue of the lesion part can effectively be collected. The lesional tissue or the like scraped off as described above is held between the outer surface of the ultrasound probe 30 and the cell collection link portions 11Da. Accordingly, the treatment instrument 1D is pulled out with the push rod handle 27 fixed. Consequently, the lesional tissue can be collected.

As described above, the second embodiment enables provision of effects that are similar to those of the above-described first embodiment. In addition to such effects, the present embodiment further enables reliable collection of lesional cells under ultrasound observation using the ultrasound probe 30. Accordingly, the present embodiment can contribute to diagnostic yield enhancement. Also, in the present embodiment, also, cells can be collected from a lesion part even if the lesion part does not extend to, for example, a lumen of a bronchus, and thus, trouble and time for searching for the bronchus involved in the lesion can be reduced. Accordingly, the present embodiment largely contributes to reduction in burden of a surgeon and reduction in pain of a patient.

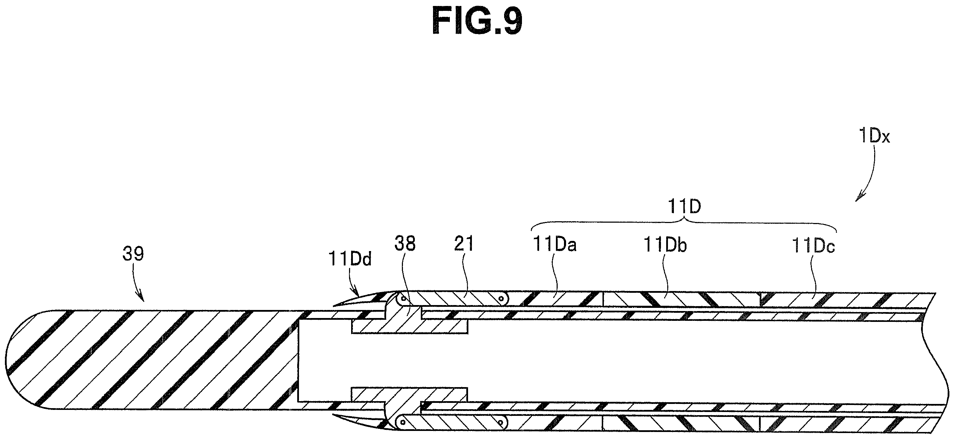

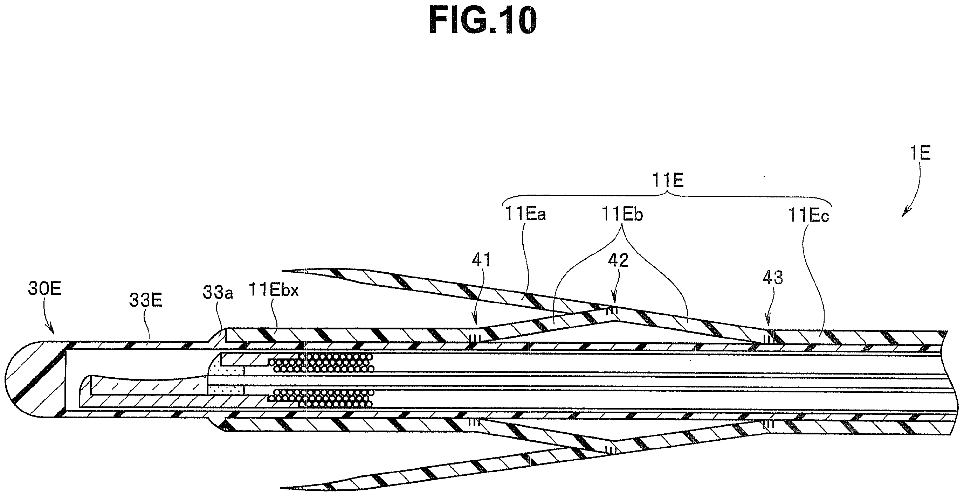

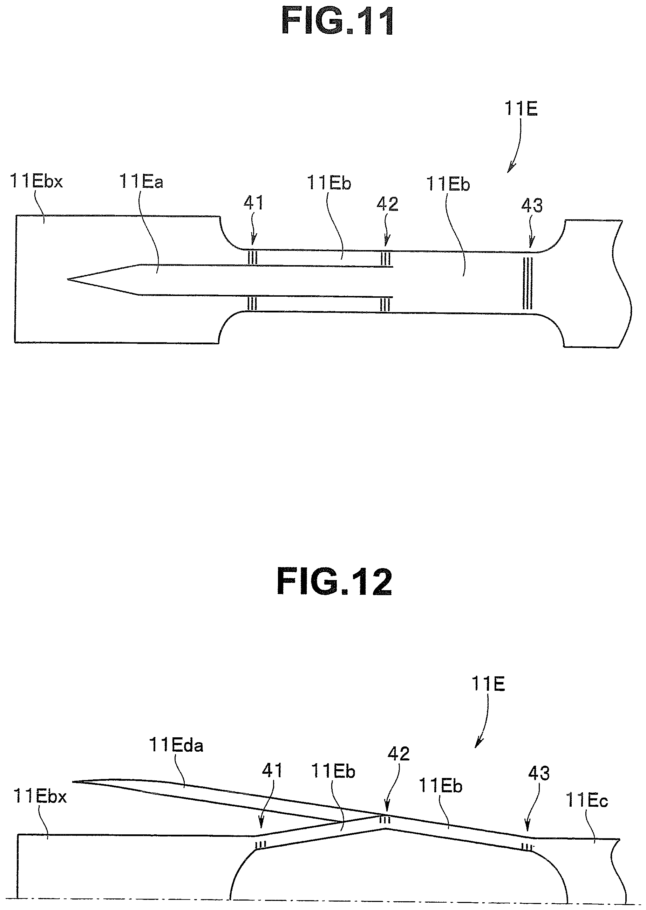

Note that, although in the present embodiment, the ultrasound probe 30 is used as a part corresponding to the insertion portion in the first embodiment, the present invention is not limited to this form. For example, as in a treatment instrument 1Dx according to a modification, which is illustrated in Fig. 9, a form in which a rod-like member 39 is provided instead of the ultrasound probe may be employed. In this case, the form is substantially similar to that of the above-described first instrument. Figs. 10 to 12 are diagrams illustrating a further embodiment of the present invention. From among the Figures, Fig. 10 is a cross-sectional diagram illustrating an overview of a configuration of a treatment instrument of the present embodiment. Figs. 11 and 12 are a top view and a side view of only a sample collection portion extracted from the treatment instrument according to the present embodiment. Note that Fig. 12 indicates a state in which a link arm is flexed and a cell collection link portion has risen.

A basic configuration of a treatment instrument 1E according to the present embodiment is substantially similar to that of the above-described previous embodiment, and is somewhat different in the configuration of sample collection portions 11E from that of the above-described embodiment. Accordingly, a detailed description of components that are similar to those of the previous embodiment will be omitted and only components that are different from those of the second embodiment will be described below.

As illustrated in Fig. 10, the treatment instrument 1E according to the present embodiment is an example in which an ultrasound probe 30E and a plurality of (two in the present embodiment) sample collection portions 11E are integrally formed.

Here, the ultrasound probe 30E has a configuration that is substantially similar to that employed in the above-described embodiment, and is somewhat different only in configuration of a distal end cap 33E from that employed in the above-described embodiment.

In other words, at the distal end cap 33E of the ultrasound probe 30E in the treatment instrument 1E according to the present embodiment, a flange shape forming portion 33a that is in contact with most distal end portions of the sample collection portions 11E and restricts the sample collection portions 11E from moving forward in an axial direction is formed. The flange shape forming portion 33a is formed in a flange shape on an outer circumferential face of the distal end cap 33E, and is a part that as a result of the most distal end portion of (a ring shape forming portion 11Ebx, which will be described later) of each sample collection portion 11E coming into contact with the flange shape forming portion 33a, fixedly supports the sample collection portions 11E on the ultrasound probe 30E. The rest of the configuration of the ultrasound probe 30E is entirely similar to that of the ultrasound probe 30 according to the above-described embodiment.

Each of the sample collection portions 11E in the treatment instrument 1E according to the present embodiment can be formed by forming a cell collection link portion 11Ea, a link arm 11Eb and a push rod 11Ec by subjecting, for example, a single-piece plate, for example, a resin tube or a metal pipe to a laser cutting processing.

As in the above-described second embodiment, most distal end portions of each cell collection link portions 11Ea is formed to be sharp and have a shape that bends toward the inside. Each link arm 11Eb includes three advancing/retracting portions 41, 42 and 43, a ring shape forming portion 11Ebx is provided so as to be continuous with the distal end side of the link arm 11Eb via the advancing/retracting portion 41, and a push rod 11Ec is provided so as to be continuous with the rear end side of the link arm 11Eb via the advancing/retracting portion 43. Here, the ring shape forming portion 11Ebx is a component portion that is a substitute for the sleeve 38 in the above-described second embodiment, and is a part that supports an end of the link arm 11Eb.

The advancing/retracting portion 41 is formed at a distal end of the link arm 11Eb, the advancing/retracting portion 43 is formed at a rear end of the link arm 11Eb, and the advancing/retracting portion 42 is formed at a proximal end portion of the cell collection link portion 11Ea, respectively. Each of the advancing/retracting portions 41, 42 and 43 is formed by, for example, performing processing to form a groove, changing a material or performing processing for reduction in thickness.

Note that the ring shape forming portion 11Ebx of each sample collection portion 11E in the present embodiment is disposed along an outer circumferential face of the ultrasound probe 30E to fixedly hold the sample collection portion 11E to prevent movement in a radial direction and fixedly hold the sample collection portion 11E to prevent forward movement in the axial direction as a result of the ring shape forming portion 11Ebx coming in contact with the flange shape forming portion 33a in the axial direction. In a state in which the sample collection portions 11E are attached to the outer circumferential face of the ultrasound probe 30E, the sample collection portions 11E may be fixed to prevent movement in the axial direction and a pivoting direction, or forward movement in the axial direction of the sample collection portions 11E may be restricted by the flange shape forming portion 33a of the ultrasound probe 30E and rearward movement in the axial direction and movement in the pivoting direction of the sample collection portions 11E may be allowed.

An operation of the treatment instrument 1E according to the present embodiment configured as described above is substantially similar to that of the above-described previous embodiment. In other words, in a normal state, the ring shape forming portion 11Ebx, the cell collection link portion 11Ea, the link arm 11Eb and the push rod 11Ec are continuously provided in this order from the distal end side, and disposed in parallel along the outer circumferential face of the ultrasound probe 30, thereby forming a linear shape in an entirety. In this state, when a push rod handle of an operation portion (not illustrated; see Fig. 7) is pushed in, the most distal end portions of the sample collection portions 11E come into contact with the flange shape forming portion 33a, whereby forward movement in the axial direction is restricted. Although a force in a direction in which the push rod 11Ec is pushed in is transmitted to the link arm 11Eb, since forward movement in the axial direction is restricted as described above, each of the advancing/retracting portions 41, 42 and 43 of the link arm 11Eb is flexed, whereby the link arm 11Eb rises. Consequently, each of the cell collection link portions 11Ea extends in a direction in which the cell collection link portion 11Ea projects in a radial direction of the ultrasound probe 30 while moving forward. The rest of the operation is substantially similar to that of the above-described second embodiment.

As described above, this embodiment enables provision of effects that are similar to those of the above-described previous embodiment. In addition, the present embodiment enables simplification of the configuration of each sample collection portion 11E so as to form each sample collection portion 11E by processing a single member, which can contribute to reduction in manufacturing costs.

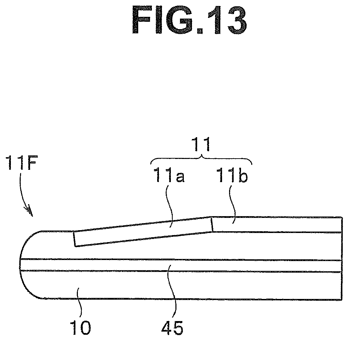

Furthermore, the third embodiment can contribute to reduction in outer diameter of the treatment instrument 1E itself, and thus, facilitates access to a lesion part such as a small-diameter periphery, enabling expansion of the possibility of sample collection. In a treatment instrument 11F according to a modification, which is illustrated in Fig. 13, a form in which an optical observation section that provides an optical field of view, for example, an image guide 45, is provided instead of the ultrasound probe may be employed.

Note that the present invention is not limited to the above-described embodiments, and it should be understood that various modifications and applications are possible without departing from the scope of the invention as described in the appended claims. Also, each of the embodiments of the present invention indicates an example of a sample collection portion, and a plurality of sample collection portions may exist around an entire circumference of a treatment instrument.

## Claims

1. A treatment instrument (1D, 1E) comprising:
a rod-like ultrasound probe (30, 30E) to be inserted into a subject, for observing inside the subject;
at least one sample collection portion (11D, 11E) arranged on a side face of the ultrasound probe (30, 30E); and
an operation portion (25) that operates the sample collection portion (11D, 11E),
wherein the sample collection portion (11D, 11E) includes,
a link portion (11Da, 11Ea) including an end supported on the ultrasound probe (30, 30E) so that the end serves as a rotation axis and the other end is pivotable relative to the rotation axis;
an advancing/retracting portion (11Db, 41, 43) including an end joined to the other end of the link portion (11Da, 11Ea), the advancing/retracting portion (11Db, 41, 43) advancing/retracting in an axial direction of the ultrasound probe (30, 30E), whereby the other end of the link portion (11Da, 11Ea) pivots relative to the rotation axis, and
a push rod (11Dc, 11Ec) arranged so that the push rod (11Dc, 11Ec) can advance/retract along the axial direction of the ultrasound probe (30, 30E), the push rod (11Dc, 11Ec) including an end joined to the other end of the advancing/retracting portion (11Db, 41, 43), and the other end joined to the operation portion (25);
wherein when the advancing/retracting portion (11Db, 41, 43) is located on a most proximal end side, the advancing/retracting portion (11Db, 41, 43) and the link portion (11Da, 11Ea) are connected in the axial direction and thereby become parallel to the ultrasound probe (30, 30E), and when the advancing/retracting portion (11Db, 41, 43) is located on a most distal end side, a part of the advancing/retracting portion (11Db, 41, 43) and the link portion (11Da, 11Ea) overlap and thereby become parallel to the ultrasound probe (30, 30E); and
wherein when the advancing/retracting portion (11Db, 41, 43) is located between a proximal end portion side and a distal end portion side, the link portion (11Da, 11Ea) is pushed out in a direction crossing the axial direction of the ultrasound probe (30, 30E) by the advancing/retracting portion (11Db, 41, 43), and thereby projects from the ultrasound probe (30, 30E).

2. The treatment instrument (1D) according to claim 1, wherein at a distal end of the advancing/retracting portion (11Db), a scraping portion (11Dd) that projects from the distal end of the advancing/retracting portion (110b) is arranged in order to scrape a target site.

3. The treatment instrument (1D, 1E) according to claim 1,
wherein the sample collection portion (11D, 11E) includes a single-piece plate; and
a part of connection between the link portion (11Da, 11Ea) and the advancing/retracting portion (11Db, 41, 43) is more flexible than another part of the sample collection portion (11D, 11E).

4. The treatment instrument according to claim 1, wherein a recess portion that is a depression for specimen collection is formed at an outer surface of the link portion.

5. The treatment instrument according to claim 1, wherein a brushlike brush portion for specimen collection is arranged at an outer side of the link portion.

6. The treatment instrument (1D, 1E) according to claim 1, wherein the other end of the link portion (11Da, 11Ea) is arranged on the distal end side relative to a distal end face of the ultrasound probe (30, 30E) when the advancing/retracting portion (11Db, 41, 43) is located on the most distal end side.

7. The treatment instrument (1D, 1E) according to claim 1, comprising an ultrasound transmission/reception section (31) that transmits/receives ultrasound in a direction perpendicular to the axial direction, at a distal end of the ultrasound probe (30, 30E).

8. The treatment instrument (1D, 1E) according to claim 1, comprising a link arm (21, 11Eb) including an end pivotably attached to the ultrasound probe and the other end pivotably attached to the link portion (11Da, 11Ea).

## Patentansprüche

1. Behandlungsinstrument (1D, 1E), das umfasst:
eine stabähnliche Ultraschallsonde (30, 30E), die in ein Individuum einzuführen ist, um das Innere des Individuums zu beobachten;
zumindest einen Probensammelabschnitt (11D, 11E), der auf einer Seitenfläche der Ultraschallsonde (30, 30E) angeordnet ist; und
einen Betriebsabschnitt (25), der den Probensammelabschnitt (11D, 11E) betreibt,
wobei der Probensammelabschnitt (11D, 11E) umfasst:
einen Verbindungsabschnitt (11Da, 11Ea), der ein Ende umfasst, das auf der Ultraschallsonde (30, 30E) getragen wird, so dass das Ende als Drehachse dient, und das andere Ende in Bezug auf die Drehachse schwenkbar ist;
einen Vorschub-/Rückziehabschnitt (11Db, 41, 43), der ein Ende umfasst, das mit dem anderen Ende des Verbindungsabschnitts (11Da, 11Ea) verbunden ist, wobei sich der Vorschub-/Rückziehabschnitt (11Db, 41, 43) in einer Axialrichtung der Ultraschallsonde (30, 30E) vorschiebt/rückzieht, wodurch das andere Ende des Verbindungsabschnitts (11Da, 11Ea) in Bezug auf die Drehachse schwenkt, und
eine Schubstange (11Dc, 11Ec), die so ausgelegt ist, dass die Schubstange (11Dc, 11Ec) sich entlang der Axialrichtung der Ultraschallsonde (30, 30E) vorschieben/zurückziehen kann, wobei die Schubstange (11Dc, 11Ec) ein Ende umfasst, das mit dem anderen Ende des Vorschub-/Rückziehabschnitts (11Db, 41, 43) verbunden ist, und das andere Ende mit dem Betriebsabschnitt (25) verbunden ist;
wobei, wenn sich der Vorschub-/Rückziehabschnitt (11Db, 41, 43) auf einer proximalsten Endseite befindet, der Vorschub-/Rückziehabschnitt (11Db, 41, 43) und der Verbindungsabschnitt (11Da, 11Ea) in Axialrichtung verbunden sind und damit parallel zur Ultraschallsonde (30, 30E) werden, und wenn sich der Vorschub-/Rückziehabschnitt (11Db, 41, 43) auf einer distalsten Endseite befindet, ein Teil des Vorschub-/Rückziehabschnitts (11Db, 41, 43) und des Verbindungsabschnitts (11Da, 11Ea) einander überlappen und somit parallel zur Ultraschallsonde (30, 30E) werden; und
wobei, wenn sich der Vorschub-/Rückziehabschnitt (11Db, 41, 43) zwischen einer proximalen Endabschnittseite und einer distalen Endabschnittseite befindet, der Verbindungsabschnitt (11Da, 11Ea) vom Vorschub-/Rückziehabschnitt (11Db, 41, 43) in einer Richtung quer zur Axialrichtung der Ultraschallsonde (30, 30E) herausgeschoben wird und somit aus der Ultraschallsonde (30, 30E) vorsteht.

2. Behandlungsinstrument (1D) nach Anspruch 1, wobei an einem distalen Ende des Vorschub-/Rückziehabschnitts (11Db) ein Schababschnitt (11Dd) angeordnet ist, der vom distalen Ende des Vorschub-/Rückziehabschnitts (11Db) vorsteht, um eine Zielstelle abzuschaben.

3. Behandlungsinstrument (1D, 1E) nach Anspruch 1,
wobei der Probensammelabschnitt (11D, 11E) eine einstückige Platte umfasst; und
ein Teil der Verbindung zwischen dem Verbindungsabschnitt (11Da, 11Ea) und dem Vorschub-/Rückziehabschnitt (11Db, 41, 43) flexibler als ein anderer Teil des Probensammelabschnitts (11D, 11E) ist.

4. Behandlungsinstrument nach Anspruch 1, wobei ein Ausnehmungsabschnitt, der eine Vertiefung zum Probensammeln ist, an einer Außenfläche des Verbindungsabschnitts gebildet ist.

5. Behandlungsinstrument nach Anspruch 1, wobei ein bürstenähnlicher Abschnitt zur Probensammlung an einer Außenseite des Verbindungsabschnitts angeordnet ist.

6. Behandlungsinstrument (1D, 1E) nach Anspruch 1, wobei das andere Ende des Verbindungsabschnitts (11Da, 11Ea) an der distalen Endseite in Bezug auf eine distale Endfläche der Ultraschallsonde (30, 30E) angeordnet ist, wenn sich der Vorschub-/Rückziehabschnitt (11Db, 41, 43) auf der distalsten Endseite befindet.

7. Behandlungsinstrument (1D, 1E) nach Anspruch 1, das einen Ultraschallübertragungs-/Ultraschallempfangsabschnitt (31) umfasst, der Ultraschall in einer Richtung lotrecht zur Axialrichtung an einem distalen Ende der Ultraschallsonde (30, 30E) sendet/empfängt.

8. Behandlungsinstrument (1D, 1E) nach Anspruch 1, das einen Verbindungsarm (21, 11Eb) umfasst, der ein Ende umfasst, das an der Ultraschallsonde schwenkbar angebracht ist, und wobei das andere Ende am Verbindungsabschnitt (11Da, 11Ea) schwenkbar angebracht ist.

## Revendications

1. Instrument de traitement (1D, 1E) comprenant :
une sonde à ultrasons de type tige (30, 30E) destinée à être introduire dans un sujet, pour observer à l'intérieur du sujet ;
au moins une partie de collecte d'échantillon (11D, 11E) disposée sur une face latérale de la sonde à ultrasons (30, 30E) ; et
une partie d'actionnement (25) qui actionne la partie de collecte d'échantillon (11D, 11E),
la partie de collecte d'échantillon (11D, 11E) comprenant :
une partie de liaison (11Da, 11Ea) comprenant une extrémité supportée sur la sonde à ultrasons (30, 30E) de telle sorte que l'extrémité sert d'axe de rotation et que l'autre extrémité est apte à pivoter par rapport à l'axe de rotation ;
une partie d'avancée/rétractation (11Db, 41, 43) comprenant une extrémité reliée à l'autre extrémité de la partie de liaison (11Da, 11Ea), la partie d'avancée/rétractation (11Db, 41, 43) avançant/se rétractant dans une direction axiale de la sonde à ultrasons (30, 30E), l'autre extrémité de la partie de liaison (11Da, 11Ea) pivotant par rapport à l'axe de rotation, et
une tige de poussée (11Dc, 11Ec) disposée de telle sorte que la tige de poussée (11Dc, 11Ec) peut avancer/se rétracter le long de la direction axiale de la sonde à ultrasons (30, 30E), la tige de poussée (11Dc, 11Ec) comprenant une extrémité reliée à l'autre extrémité de la partie d'avancée/rétractation (11Db, 41, 43), et l'autre extrémité reliée à la partie d'actionnement (25) ;
dans lequel, lorsque la partie d'avancée/rétractation (11Db, 41, 43) est située sur un côté extrémité la plus proximale, la partie d'avancée/rétractation (11Db, 41, 43) et la partie de liaison (11Da, 11Ea) sont reliées dans la direction axiale et, de ce fait, deviennent parallèles à la sonde à ultrasons (30, 30E) et, lorsque la partie d'avancée/rétractation (11Db, 41, 43) est située sur un côté extrémité la plus distale, une partie de la partie d'avancée/rétractation (11Db, 41, 43) et la partie de liaison (11Da, 11Ea) se chevauchent et, de ce fait, deviennent parallèles à la sonde à ultrasons (30, 30E) ; et
dans lequel, lorsque la partie d'avancée/rétractation (11Db, 41, 43) est située entre un côté partie d'extrémité proximale et un côté partie d'extrémité distale, la partie de liaison (11Da, 11Ea) est poussée à l'extérieur dans une direction coupant la direction axiale de la sonde à ultrasons (30, 30E) par la partie d'avancée/rétractation (11Db, 41, 43) et, de ce fait, se projette à partir de la sonde à ultrasons (30, 30E).

2. Instrument de traitement (1D) selon la revendication 1, dans lequel, à une extrémité distale de la partie d'avancée/rétractation (11Db), une partie de grattage (11Dd) qui se projette à partir de l'extrémité distale de la partie d'avancée/rétractation (11Db) est disposée afin de gratter un site cible.

3. Instrument de traitement (1D, 1E) selon la revendication 1,
dans lequel la partie de collecte d'échantillon (11D, 11E) comprend une plaque d'un seul tenant ; et
une portion du raccordement entre la partie de liaison (11Da, 11Ea) et la partie d'avancée/rétractation (11Db, 41, 43) est plus souple qu'une autre portion de la partie de collecte d'échantillon (11D, 11E).

4. Instrument de traitement selon la revendication 1, dans lequel une partie d'évidement qui est un creux pour la collecte d'échantillon est formée sur une surface externe de la partie de liaison.

5. Instrument de traitement selon la revendication 1, dans lequel une partie brosse de type brosse pour la collecte d'échantillon est disposée à un côté externe de la partie de liaison.

6. Instrument de traitement (1D, 1E) selon la revendication 1, dans lequel l'autre extrémité de la partie de liaison (11Da, 11Ea) est disposée sur le côté extrémité distale par rapport à une face d'extrémité distale de la sonde à ultrasons (30, 30E) lorsque la partie d'avancée/rétractation (11Db, 41, 43) est située sur le côté extrémité la plus distale.

7. Instrument de traitement (1D, 1E) selon la revendication 1, comprenant une section d'émission/réception d'ultrasons (31) qui émet/reçoit des ultrasons dans une direction perpendiculaire à la direction axiale, au niveau d'une extrémité distale de la sonde à ultrasons (30, 30E).

8. Instrument de traitement (1D, 1E) selon la revendication 1, comprenant un bras de liaison (21, 11Eb) comprenant une extrémité fixée de manière pivotante à la sonde à ultrasons et l'autre extrémité fixée de manière pivotante à la partie de liaison (11Da, 11Ea).
